# EUROPEAN PATENT SPECIFICATION

(11) **EP 0 876 161 B1**
(45) Date of publication and mention of the grant of the patent: **21.11.2001**
(21) Application number: 97901180.6
(22) Date of filing: 23.01.1997
(51) Int. Cl.: A61K 49/04, A61K 49/00

(54) **CONTRAST MEDIA**
KONTRASTMITTEL
SUBSTANCES DE CONTRASTE

(30) Priority: 23.01.1996 GB 9601340
(43) Date of publication of application: 11.11.1998
(73) Proprietor: NYCOMED SALUTAR, INC., Wayne, PA 19087-8630 (US)
(72) Inventor: DROEGE, Michael, Livermore, CA 94550 (US); YU, Shi-Bao, Malvern, PA 19355 (US); SANDERSON, William, Wayne, PA 19087 (US); BACON, Edward, Audubon, PA 19403 (US); DELECKI, Daniel, Radnor, PA 19087 (US); EARLEY, William, Spring City, PA 19475 (US); YE, Naidong, Norristown, PA 19403 (US)
(74) Representative: Hammett, Audrey Grace Campbell
(86) International application number: GB9700211
(87) International publication number: WO9726921

(56) References cited:
- EP-A- 0 645 146
- WO-A-91/14460
- WO-A-92/17215
- WO-A-93/02713
- WO-A-97/03993
- WO-A-97/03994
- WO-A-97/05904
- SCHMIDT S ET AL: "OCTAHEDRAL METALLO-MESOGENS OF CHROMIUM, MOLYBDENUM AND TUNGSTEN WITH 1,4,7-TRISUBSTITUTED 1,4,7-TRIAZACYCLONONANE AND THREE CARBONYL GROUPS AS LIGANDS" LIQUID CRYSTALS, vol. 16, no. 4, 1 April 1994, pages 693-702, XP000434157

## Description

The present invention relates to the use in diagnostic imaging, in particular X-ray, ultrasound and scintigraphy of contrast agents comprising complexes of multinuclear moieties, and to contrast media containing such complexes.

All diagnostic imaging is based on the achievement of different signal levels from different structures within the body. Thus in X-ray imaging for example, for a given body structure to be visible in the image, the X-ray attenuation by that structure must differ from that of the surrounding tissues. The difference in signal between the body structure and its surroundings is frequently termed contrast and much effort has been devoted to means of enhancing contrast in diagnostic imaging since the greater the contrast between a body structure and its surroundings the higher the quality of the images and the greater their value to the physician performing the diagnosis. Moreover, the greater the contrast the smaller the body structures that may be visualized in the imaging procedure, i.e. increased contrast can lead to increased spatial resolution.

The diagnostic quality of images is strongly dependent on the inherent noise level in the imaging procedure - and the ratio of the contrast level to the noise level can thus be seen to represent an effective diagnostic quality factor for diagnostic images.

Achieving improvement in such a diagnostic quality factor has long been and still remains an important goal. In techniques such as X-ray and ultrasound, one approach to improving the diagnostic quality factor has been to introduce contrast enhancing materials, contrast agents, into the body region being imaged.

Thus in X-ray for example early examples of contrast agents were insoluble inorganic barium salts which enhanced X-ray attenuation in the body zones into which they distributed. More recently the field of X-ray contrast agents has been dominated by soluble iodine containing compounds such as those marketed by Nycomed AS under the trade names Omnipaque and Amipaque.

Much recent work on X-ray contrast agents has concentrated on aminopolycarboxylic acid (APCA) chelates of heavy metal ions and, recognising that effective imaging of many body sites requires localization at the body sites in question of relatively high concentrations of the metal ions, there have been suggestions that polychelants, that is substances possessing more than one separate chelant moiety, might be used to achieve this.

More recently it has been found that contrast enhancement may be achieved particularly effectively by the use of multinuclear complexes, that is complexes wherein the complexed moiety itself comprises two or more contrast enhancing atoms. Thus, for X-ray or ultrasound the complex would comprise two or more heavy metal atoms and for MRI the complex would contain two or more metal atoms with paramagnetic properties.

However, we have now found that multinuclear complexes comprising two ligand-conjugated multinuclear clusters are particularly effective diagnostic imaging contrast enhancers, in particular for X-ray imaging.

Thus, viewed from one aspect the present invention provides a diagnostic imaging contrast medium comprising an image contrast enhancing physiologically tolerable complex, said complex comprising a pair of interconjugated multinuclear clusters, or a physiologically tolerable salt thereof, together with at least one pharmaceutical carrier or excipient.

By "multinuclear cluster" is meant a covalently bound molecule or ion containing at least two, and preferably 3, 4, 5 or 6, especially 3 or 4 metal atoms, in particular heavy metals or paramagnetic metals or metals having radiation emitting isotopes. Clusters containing heavy metals (ie. row 5 of the periodic table or higher) and in particular W or Mo, especially W, are particularly preferred, especially W₃ and W₄ clusters.

In such multinuclear clusters, besides the contrast enhancing atoms, generally metal atoms, the cluster framework will generally include other atoms which may for example serve as bridges between the contrast enhancing atoms. Such further atoms include for example oxygen and sulphur and thus many multinuclear clusters will be in the form of polyoxo cations and the full and partial sulfur analogues.

The dimeric cluster complexes, eg. those in which the individual clusters contain three contrast enhancing atoms M, ie. (M₃)₂ complexes, have particular potential as contrast agents since, relative to simple monomeric multinuclear complexes, the increase in the contrast enhancing atom content of the molecule is achieved with little increase in the volume occupied by the contrast agent complex. Thus the use of such complexes enables a high ratio of contrast enhancing atom to overall complex volume to be achieved. By increasing the relative content of contrast enhancing atoms in this way the total quantity of the contrast agent to achieve the same contrast effect may be reduced and problems associated with contrast agent solubility or toxicity or with contrast medium viscosity may also be reduced.

Viewed from another aspect the invention provides the use of a physiologically tolerable complex, said complex comprising a pair of interconjugated multinuclear clusters, or a physiologically tolerable salt thereof, for the manufacture of a contrast medium composition for use in imaging of the human or non-human animal body.

Viewed from a still further aspect, the invention provides a method of generating an image of a human or non-human animal, preferably mammalian, body which method comprises administering to said body a contrast enhancing amount of a physiologically tolerable complex, said complex comprising a pair of interconjugated multinuclear clusters, or a physiologically tolerable salt thereof, and generating an image of at least part of said body into which said agent distributes.

Preferred multinuclear complexes for use in accordance with the invention include those having two M₃ clusters (which may of course include other atoms besides the three contrast enhancing atoms M) linked together by three chelant groups L, each of which coordinates to both clusters. This can be expressed by the general formula I

(M₃)₂ L₃ (I)

Particularly preferred complexes for use in contrast media according to the invention include those in which M is selected from the group VIb metals, in particular those in which M is Mo and/or W, especially preferably those in which each M is W.

As mentioned above the multinuclear clusters may also contain further atoms which may have little or no contrast enhancing effect but which may for example function as bridging atoms bonding the contrast enhancing atoms in each M₃ cluster together. Particularly suitable examples of such bridging atoms include the atoms of groups VIa and VIIa, eg. oxygen, sulphur, selenium, tellurium and halogen atoms.

Each M₃ cluster may thus, for example, be present as a group of formula X₃SₐO_{b} where a is 1, 2, 3 or 4, b is 0, 1, 2 or 3, a+b is 4, and X is a metal atom.

Particularly preferably, the M₃ clusters may be present as W₃S₄ [ie. W₃(*µ*₃S) (*µ*₂S)₃] or W₃SO₃.

The symbols "*µ*₃S" and "*µ*₂S" here indicate sulphur atoms bound respectively to 3 and 2 metal atoms in the cluster.

Besides the ligands which interlink the multinuclear clusters, the complexes may of course include further ligands which coordinate only to a single cluster.

The ligands, either those which interlink or those which coordinate to a single cluster, can be selected from a wide range of structures. The range of metal coordinating and cluster coordinating ligands is very broad and is described at length in the scientific and patent literature relating to heavy metal detoxification and sequestering and to chelate based magnetic resonance imaging contrast agents. In the latter regard the reader is referred to the numerous patent publications of Schering, Nycomed Imaging, Nycomed Salutar, Squibb, Mallinckrodt, Bracco and Guerbet.

For interlinking clusters, the ligands L may conveniently represent linear, branched or cyclic polyamino, polyaminocarboxylic or polycarboxylic acids. More specifically, L may be represented by the formula II:

(R²)₂N[(CHR⁴)ₘNR¹]ₙ(CHR⁴)ₘN(R²)₂ (II)

where each R¹ which may be the same or different represents a group R² or a C₁₋₄alkyl, phenyl-C₁₋₄alkyl, C₁₋₄hydroxyalkyl or amino-C₁₋₄ alkyl group or two R¹ groups may together represent a group CH₂CH₂NR³CH₂CH₂ where R³ is an R² group or a C₁₋₄ alkyl group optionally substituted by hydroxyl, carboxyl, aryl or amino groups; n is 0, 1 or 2; each m is 2, 3 or 4 preferably 2; and each R⁴ denotes a group R¹ or a carboxyl, hydroxyl or C₁₋₄alkoxy group; and each R² independently represents a hydrogen atom or an optionally amidated or esterified carboxy - (C₁₋₄ alkyl) group, wherein any amide nitrogen is substituted by group selected from hydrogen atoms and optionally hydroxylated C₁₋₄ alkyl groups; preferably optionally hydroxylated C₁₋₄ alkyl groups; preferably such that an R⁴ or R¹ group contains a quaternary amine group.

Particularly preferably L is represented by the formulae III, IV or V

(R²)₂NCH₂CH₂NR¹CH₂CH₂NR¹CH₂CH₂N (R²)₂ (III)

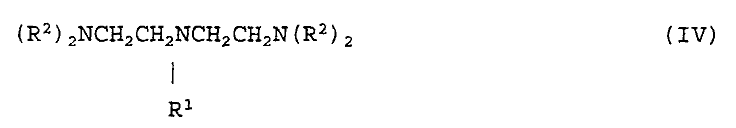

(R²)₂NCH₂CHR⁴N(R²)₂ (V)

in which R¹, R² and R⁴ are as defined above.

For example, L may be selected from (HOOCCH₂)₂NCH₂CH₂N(CH₂CH₂OH)CH₂CH₂N(CH₂CH₂OH)CH₂CH₂N(CH₂COOH)₂ (HOCH₂CH₂)₂NCOCH₂N(CH₂COOH)CH₂CH₂(N(CH₂COOH)CH₂CH₂)₂N(CH₂COOH)CH₂CON(CH₂CH₂OH)₂ (HOOCCH₂)₂NCH₂CH(CH₃)N (CH₂COOH)₂ H₂NCH₂CH₂N(CH₂COOH)CH₂CH₂N(CH₂COOH)CH₂CH₂NH₂ (HOOCCH₂)₂NCH₂CH₂N(CH₂CH₂N(CH₂CH₂OH)₂)CH₂CH₂N(CH₂CH₂N(CH₂CH₂OH)₂)CH₂CH₂N(CH₂COOH)₂ (HOOCCH₂)₂NCH₂CH₂N(CH₂COOH)CH₂CH₂N(CH₂COOH)CH₂CH₂N(CH₂COOH)₂
and 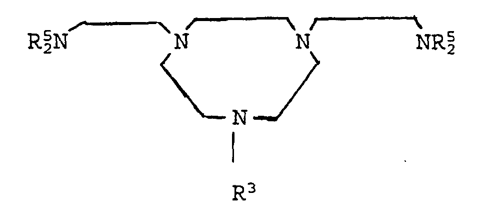 where each R⁵ is hydrogen or carboxymethyl and R³ is hydroxyalkyl or N-carboxymethylated amino alkyl.

Particularly preferably, L may be selected from (HOOCCH₂)₂NCH₂CH₂N(CH(CH₂OH)₂)CH₂CH₂N(CH₂COOH)₂ and (HOOCCH₂)₂NCH₂CH₂N (C(CH₂OH)₃)CH₂CH₂N(CH₂COOH)₂.

Further suitable ligands are shown in Table 1 below:

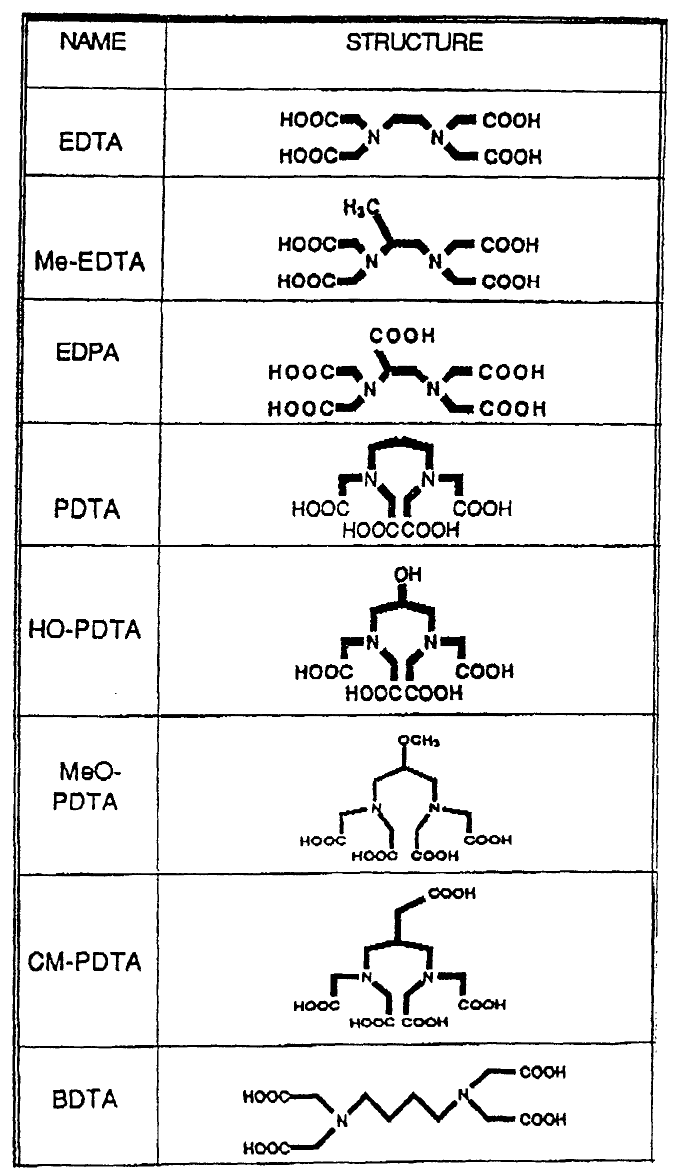

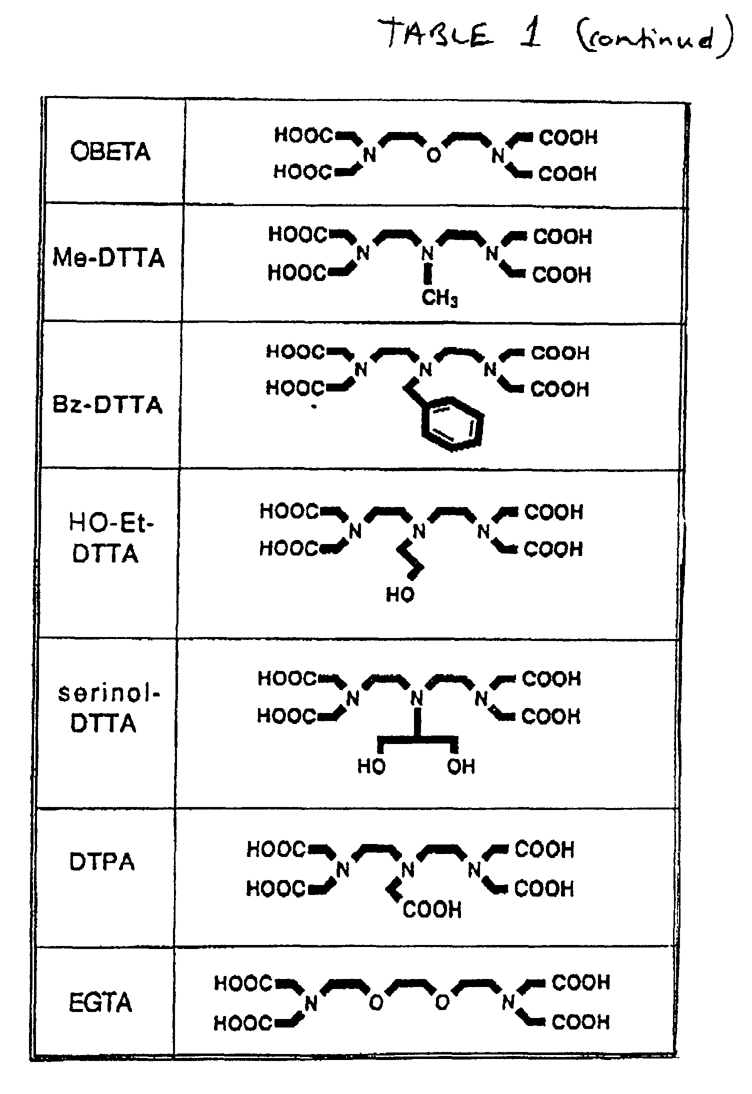

Besides the compounds of formula II, many of the most useful ligands useable according to the invention fall into the more general formula VI

Z (X (CHR⁶)ₐ)_{b}XZ (VI)

(where a is an integer of from 2 to 12, preferably 2 to 10, e.g. 2, 3, or 4; b is an integer of from 1 to 8, preferably 2, 3 or 4;
each R⁶, independently is hydrogen, a hydrophilic or charged group (e.g. a hydroxyalkyl or quaternary aminoalkyl group) or two groups R⁶, or one R⁶ and one group Z, together represent a saturated or unsaturated heterocyclic or carbocyclic ring, preferably with 5-7 ring atoms;
each X independently is O, S, NZ or PZ,
each Z indpendently is hydrogen, hydroxyalkyl, mercaptoalkyl, carboxyalkyl (or an amide or ester derivative thereof e.g. -CH₂CONHCH₃) or optionally hydroxy or mercapto substituted acyl, or is a side chain ((CHR⁶)ₐ X*)_{c}Z* (where c is 1 to 4 and X* and Z* are as defined for X and Z but do not represent any group containing a X* or Z* group) or two groups Z together form a bridging group ((CHR⁶)ₐ X*)_{c}(CHR⁶)ₐ) or are salts thereof).

While polyamines and polyethers, especially linear or cyclic polyamines and polyethers, such as ethylenediamine,1,4,7-triazacyclononane and cyclen, can be used as ligands, in general linear aminopolycarboxylic acids (APCAs) substituted to carry a pendant charged or hydrophilic group are preferred, particularly DTPA, EDTA and TTHA derivatives thereof and analogues, and other cyclic and non-cyclic APCAs as defined in WO-A-89/00557.

In the ligands of formulae II to VI described above, unless otherwise stated, any alkyl moiety preferably has a carbon atom content of up to 8, any cyclic group other than the macrocyclic skeleton of a macrocyclic ligand is preferably a three to eight membered ring and any carboxyl derivative is preferably an amide group.

The complexes used according to the invention may be ionic or, more preferably, may carry no net charge; most preferably they are non-ionic. Moreover they may be water-soluble or, less preferably, water-insoluble. Any necessary counterions should of course most preferably also be physiologically tolerable.

The range of physiologically acceptable counterions for therapeutically active agents is of course well known to pharmacologists.

Suitable countercations include for example protons, alkali and alkaline earth metal ions, e.g. sodium, calcium and magnesium and zinc, ammonium and organic cations (e.g. organic amine cations, quaternary ammonium, pyridinium, meglumine, alkylammonium, polyhydroxy-alkylammonium, basic protonated amino acids, etc), transition metal complex cations, organometallic cations, etc. Suitable counteranions include for example halide (e.g. chloride, bromide, iodide, I₃⁻ etc.), sulphate, mesylate, phosphate, etc.

The 2:3 complexes, ie. two clusters interlinked by 3 ligands, such as those of formula I above, are novel and these and their salts, especially the physiologically acceptable salts form a further aspect of the invention. In this regard, particular reference is made to (W₃SₐO_{b})₂[(R²)₂N[(CHR⁴)ₘNR¹]ₙ(CHR⁴)ₘN(R²)]₃ complexes and salts thereof.

In the complexes used in the invention, it is particularly preferred that the electrical charge carried by the ligands L should substantially if not completely balance that carried by the M₃ clusters. Thus, for example, in the case of the 2:3 complexes of W₃SO₃ it is preferably to reduce the overall negative charge inherent in such a complex whilst simultaneously increasing the water solubility/hydrophilicity of the cluster complex.

We have found that this can be achieved by the use of so-called "charge compensation" chelate ligands which carry a positive charge, preferably in the form of a quaternary ammonium group located either in the backbone of the bridging ligand or, more preferably, in a backbone substituent of the ligand. On complexation with, for example, two W₃SO₃ clusters, such "charge compensation" ligands are capable of forming a 2:3 zwitterionic complex with a lower overall ionic charge (reduced from -4 to -1).

In addition, the positive centre of such "charge compensation" ligands may be functionalised with groups such as for example hydroxyl groups, which serve to increase the solubility/hydrophilicity of the resulting complex.

Two main approaches can be used to introduce charge compensation: (i) prepare positively charged ligands (such as quaternary ammonium containing) and then form a 2:3 complex with the W₃SO₃ cluster; and (ii) start with a pre-formed 2:3 cluster/ligand complex and use a chemical reaction at a non-tungsten coordinating functional group (amine, carboxylate, etc.) to introduce positive charge (quaternary ammonium for example).

The precise location of the positive centre on the chelate ligand has been found to affect the complexation reaction pathway and in some cases may shift the pathway away from formation of the desired 2:3 complexes towards undesired polymeric materials.

Alternatively it is possible to introduce positive charge into a pre-formed 2:3 complex. This approach uses a post-complexation functionalization of a pre-formed 2:3 complex of W₃SO₃ to derivatize a portion of the ligand in a 2:3 complex that is not involved in coordinating to tungsten. Charge compensation is achieved by either inducing a positive centre (such as alkylation to form a quaternary ammonium center at an amine of the ligand that is not involved in binding to tungsten) or by attaching a cationic functional group (such as a quaternary ammonium group) to a functional group of the ligand that is not attached to tungsten. This is an attractive approach because it overcomes any possible difficulties of complexation with positively charged ligands and because straightforward organic chemistry can be used to attach different types of hydrophilic and charged groups to already formed and purified 2:3 complexes of W₃SO₃.

For example, the reaction of the 2:3 W₃SO₃ complex of DTPA and/or carboxymethyl-PDTA with a group possessing a quaternary nitrogen atom appears to be a particularly promising route, in particular reaction with cholamine or its bis(hydroxyethylated) analog N-methyl-N,N-bis(hydroxyethyl)ethylenediamine which possess an amino group capable of reacting with the carboxyl group of DTPA or carboxymethyl-PDTA and a quaternary nitrogen atom to balance the negative charges on the complex, eg. the (M₃)₂L₃ complex.

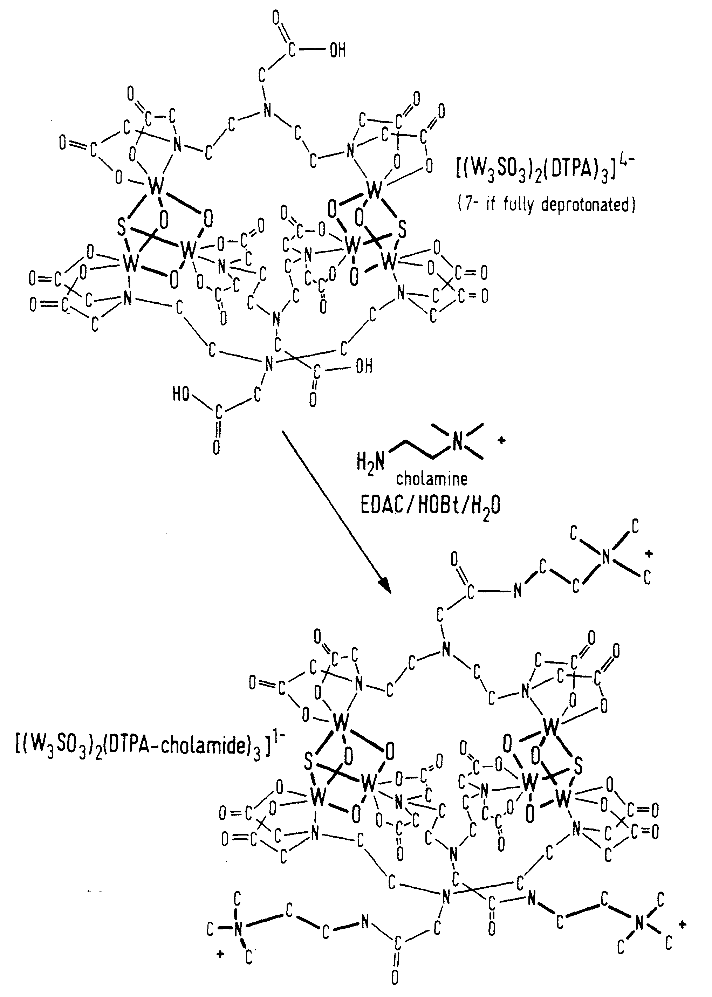

Figure 1 hereinafter illustrates the reaction of (W₃SO₃)₂(DTPA)₃ with cholamine to produce a complex having a single overall negative charge.

A further alternative approach to reducing the overall charge of the complexes for use in accordance with the invention is by coordination of suitable metal ions of appropriate charge between the incomplete cube faces of M₃ clusters. Such metal atoms may conveniently be present as a cryptated species held in postion between the M₃ clusters by coordination with the ligands present.

Thus, for example, in the case of 2 W₃SO₃ clusters, the 3 oxygen atoms on each cluster may form an octahedral coordination environment for binding of a suitably charged metal ion:

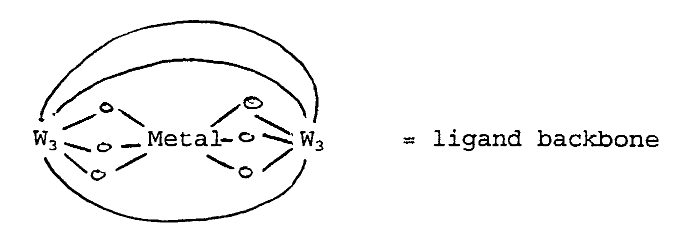

Metal ions of particular interest include those from the groups IVa and IVb and Ce(IV). Such metal ions may be present in the +4 oxidation state, resulting in a complex having an overall charge of zero. Lower charged metal ions having a charge of +3, +2 and +1 may however be used to achieve partial charge compensation.

As outlined above, metal ions may be incorporated into the cluster complex as cryptated metal ions held between the M₃ clusters rather than by being coordinated to the M₃ clusters. This can be illustrated in the case of two W₃ clusters as below:

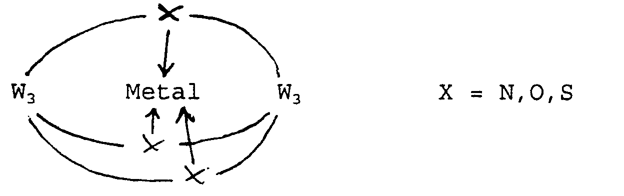

Suitable metal ions include those of groups Ia, IIa and IIIa, transition metal ions and lanthanide elements. As will be appreciated, in the case of 2:3 complex illustrated above, as the metal ion charge varies from +1 to +4, the overall charge of the complex will vary from -3 to 0.

The complexes used according to the invention may be prepared by reaction of multinuclear cluster comounds with the appropriate ligands, optionally having selected groups protected during the dimer formation reaction and with deprotection being effected thereafter. Such a reaction forms a further aspect of the present invention.

The ligands used may be ligands known from the literature or may be prepared by analogous methods to those described in the literature.

The cluster compounds used may be compounds known from the literature or may be prepared by analogous methods to those described in the literature, eg. in WO-91/14460 and WO 92/17215 and the references set out therein. A particularly useful method for the preparation of [W₃SO₃(H₂O)₉]Cl₄ comprises a variant of the process described in WO 92/17215, in which the reaction product of W(CO₆) and Na₂S is treated with at least 6N HCl, and preferably approximately 12N HCl, prior to ion exchange chromatography.

For adminstration to human or animal subjects, the complexes will conveniently be formulated in sterile form together with pharmaceutical or veterinary carriers or excipient. The contrast media of the invention may conveniently contain pharmaceutical or veterinary formulation aids, for example stabilizers, antioxidants, osmolality adjusting agents, buffers, pH adjusting agents, colorants, flavours, viscosity adjusting agents and the like. They may be in forms suitable for parenteral or enteral administration, for example, injection or infusion or administration directly into a body cavity having an external voidance duct, for example the gastrointestinal tract, the bladder and the uterus. Thus the media of the invention may be in conventional pharmaceutical adminstration forms such as tablets, coated tablets, capsules, powders, solutions, suspensions, dispersions, syrups, suppositories, emulsions, liposomes, etc; solutions, suspensions and dispersions in physiologically acceptable carrier media, e.g. water for injections, will however generally be preferred. Where the medium is formulated for parenteral administration, the carrier medium incorporating the multinuclear complex is preferably isotonic or somewhat hypertonic. Moreover, media for parenteral administration will preferably contain small quantities, e.g. 0.01 to 10 mole percent relative to the multinuclear complex of free chelants or of weak chelate complexes with physiologically tolerable chelated species (e.g. Ca²⁺); small additions of sodium or calcium salts may also advantageously be made.

For use as X-ray contrast media, the media of the invention should generally have a heavy atom content of 1 millimole/l to 5 mole/l, preferably 0.1 to 2 mole/l. Dosages of from 0.05 to 2.0 mmoles/Kg, e.g. 0.5 to 1.5 mmoles/kg will generally be sufficient to provide adequate contrast although dosages of 0.8 to 1.2 mmoles/kg will normally be preferred.

For scintigraphy, dosages of the radioactive species will generally be lower.

The present invention will now be described by way of the following Examples, in which all percentages and ratios are by weight and all temperatures are in degrees Celsius, unless otherwise specified.

### EXAMPLE 1

### Preparation of oxybis(ethyliminodiacetic acid) (OBETA)

Chloroacetic acid (13.3g, 141 mmol) was dissolved in 15 ml 30% NaOH. To this was added 2,2'-oxybis(ethylamine)-dihydrochloride (5.0g, 28.2 mmol) with stirring.
Further 30% NaOH solution was added to keep the pH between 10 and 11.5 during the course of the reaction. After 2½ hours the reaction was heated to 40°, and then to 90° after a further 3 hours. The reaction was left overnight at ambient temperature, and then heated for 12 hours at 90°, during which time it consumed only a small amount of NaOH. ¹H-NMR indicated the reaction to be completed at this stage. The product was then loaded onto an AG1-X8 acetate form ion exchange resin column and eluted with 4-5N HOAc. The non-bound portion of the product was mixed with excess sodium acetate, loaded onto an AG50W H⁺ column, and eluted with NH₄OH. The fractions containing clean product from both columns were combined, basified and rechromatographed through AG1-X8 acetate form ion exchange resin to give 6g (63%) of a white foam product on drying under vacuum.
¹³C NMR (D₂O) 55.3, 57.4, 65.0, 170.0.
¹H NMR (D₂O) 3.42, t, 4H; 3.67, t, 4H; 3.89, s, 8H.

| | | | |
|---|---|---|---|
| Calc OBETA + 0.1 H₂O | 42.63% C, | 6.02% H, | 8.29% N. |
| Found | 42.54% C, | 5.94% H, | 8.34% N. |

### EXAMPLE 2

### Preparation of serinol DTTA

2-Bromoethyl-iminodiacetic acid-t-butyl ester (36.4g, 103 mmol), serinol (4.28g, 47 mmol) and trisodium phosphate (77g, 470 mmol) were combined in 200 ml acetonitrile and heated to reflux overnight with stirring. The next day, TLC of the solution showed one major spot and three very minor spots. The mixture was filtered and the filter cake washed with further CH₃CN and discarded. The filtrate was evaporated to dryness to give 36g of a thick yellow oil, which was then diluted with methanol and treated with 6N HCl (200 ml). After one hour stirring, this was evaporated to dryness and treated with further 6N HCl for an hour. The solution was evaported to dryness, and chased once with water to give 34g solid (¹H-NMR indicated ∼98% complete deprotection). This was dissolved in water and the pH raised to 11 with NaOH. The product was loaded onto 800 cm³ AG1-X8 OH-form ion exchange resin, washed with water, and eluted with acetic acid. Pure desired product was eluted off with 3N acetic acid, and further impure product was recovered with additional 3N and 4N acetic acid. The fractions containing pure product were combined and chased repeatedly with H₂O to give 9.2g of a white foam on drying under vacuum. The impure product was rechromatographed under the same conditions to give an additional 3g product for a total yield of 12.2g (64%).
¹³C NMR (D₂O) 50.0, 53.2, 58.1, 59.5, 66.0, 173.6.
¹H NMR (D₂O) 3.24, m, 9H; 3.56, d, 4H; 3.62, s, 8H.
C₁₅H₂₇N₃O₁₀•1.4 H₂O.

| | | | |
|---|---|---|---|
| Calc | 41.5% C, | 6.9% H, | 9.7% N. |
| Found | 41.5% C, | 6.8% H, | 9.6% N. |

TGA shows 5.1 wt% loss (ambient temperature to 150°C) for 1.2 H₂O/mol.

### EXAMPLE 3

### Preparation of DTTA-Tris

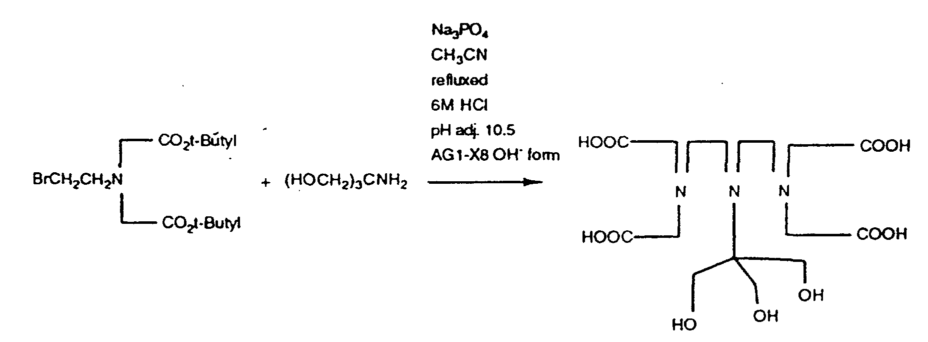

A 3 necked, 1.0 L round bottom flask equipped with an overhead stirrer, reflux condenser, oil bath and hot plate was charged with 8.278g (0.0683 moles) (HOCH₂)₃CNH₂ which was dissolved using 200 ml of 10% MeOH/CH₃CN. 123.20g (0.752 moles) Na₃PO₄, 52.97g (0.150 moles) bromoethyl-IDA, t-butyl-ester and approximately 650 ml of 10% MeOH/CH₃CN was added and the mixture was refluxed (85-90°C) for 20 hours under a N₂ purge and the reaction was followed by TLC. After 40 hours no more bromoethyl-IDA was observed. The reaction was stopped, cooled, filtered over a fine frit to remove the Na₃PO₄, rinsed with CH₃CN, reduced to dryness and 54.13g of yellow oil were collected. The t-butyl groups were removed by the addition of 100 ml conc HCl and 100 ml distilled water. After stirring for over an hour, the solution was reduced to an oil by rotary-evaporation, chased 3 times with distilled water and ¹H NMR confirmed that all of the t-butyl groups had been removed.

The yellow oil was diluted in approximately 100-200 ml of distilled water, basified using 2.0N NaOH to pH 10.5, reduced in volume, readjusted to pH 10.5, filtered over a fine frit and loaded onto an AG1-X8 column in the OH-form. The column was washed with 2.0 L of distilled water. The eluant was evaporated to dryness and ¹H NMR confirmed that the water wash contained impurities and sodium hydroxide. The column was washed with 3-4 L each of 1.0 N, 2.0 N and 3.0 N HOAc solutions and ¹H and ¹³C NMR confirmed that these washes did not contain the desired title product. The column was further washed with 10.0 L of 4.0 N HOAc and 5L of 5.0 N HOAc solutions, and following evaporation under vacuum >28g of crispy white solid (93% yield) was obtained. It was confirmed by ¹H (integration fit 8:6:8) and ¹³C (ppm; 172.9, 70.8, 63.0, 58.6, 56.0, 49.5) NMR and by mass spectroscopy. (M-H⁺ at 440 amu.) that the 4.0 N/5.0 N HOAc washes contained the desired product, DTTA-Tris.
C₆H₂₉N₃O₁₁•2 H₂O:

| | | | |
|---|---|---|---|
| Calc | C: 40.42 | H: 7.00 | N: 8.84 |
| Found | C: 40.29 | H: 6.72 | N: 8.77 |

### EXAMPLE 4

### Preparation of N'-(polyhydroxyalkyl)-N'-methyldiethylenetriaminetetraacetic acid

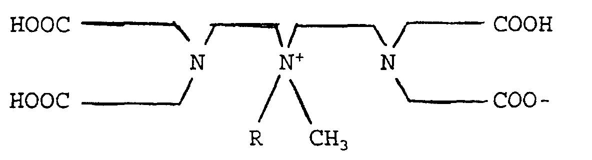

### (a) R = CH₂(CHOH)₄CH₂OH

N-methylglucamine (9.76g, 0.05M), 2-bromoethyliminodiacetic acid di-t-butyl ester (38.75g, 0.11M), anhydrous trisodium phosphate (20g, 0.12M), and acetonitrile (600 ml) were stirred and refluxed under nitrogen for 18 hours. The reaction mixture was filtered, and the solvent evaporated. The resulting pale yellow residue was treated with 6N HCl (100 ml), stirred for 1 hour to hydrolyze the ester groups, and then reevaporated to yield crude ligand (40.0g) as a pale yellow solid.

A portion (5g) of this solid was chromatographed on AG-1 X8 (acetate form) ion exchange resin. After elution with 0.75N acetic acid, the purified ligand (2.57g, representing a reaction yield of 80%) was eluted with 1N acetic acid. It could be further purified by dissolving in water and precipitation with methanol.

FAB-MS showed MH⁺ 514, calculated = 514.
¹H NMR: 4.1 ppm, t, 1H, terminal CHOH; 3.2-3.8 ppm, m (including major s at 3.5 ppm, CH₂COO), 24H (theor. = 23); 3.0 ppm, s, 3H, NCH₃.

### (b) R = CH₂CHOHCH₂OH

This ligand was prepared by the method described in (a) above using N-methylpropane-2,3-diol (5.25g, 0.05M), 2-bromoethyliminodiacetic acid di-t-butyl ester (38.75g, 0.11M), anhydrous Na₃PO₄ (20g, 0.12M), and CH₃CN (600 ml). The crude, hydrolysed product weighed 32.8g. A portion (5g) was chromatographed on AG-1 X8 (acetate) and, after elution with 0.75N acetic acid, elution with 1N acetic acid yielded purified ligand (2.98g, representing a reaction yield of 92%). It could be further purified by dissolving in water and precipitation with isopropanol.

FAB-MS showed MH⁺ 424, calculated = 424.
¹H NMR: 4.1 ppm, t, 1H, CHOH; 3.3-3.9 ppm, m (including major s at 3.6 ppm, CH₂COO), 23H (theor. = 20); 3.1 ppm, s, 3H, NCH₃.

### EXAMPLE 5

### Preparation of carboxyethylenediaminetetraacetic acid (EDPA)

Chloroacetic acid (5 equiv) was dissolved in 30% NaOH. To this was added 2,3-diaminopropionic acid (1 equiv) with stirring. Further 30% NaOH solution was added to keep the pH between 10 and 11.5 during the course of the reaction. After 2.5 hours the reaction was heated to 40°C, and then to 90°C after a further 3 hours. The reaction was left overnight at RT, and then heated for a day at 90°C, during which time it consumed only a small amount of NaOH. After this the reaction appeared complete by 1H-NMR. The product was loaded onto an AG1-X8 acetate form ion exchange resin column; the ligand was not fully purified by ion exchange chromatography, and hence was further purified by recrystallization from water.
FAB MS: MH⁺ found, 337; calculated, 337.
¹H NMR (D₂O, ppm) : 3.0(m), 2.7 (d), 2.3(m).
¹³C NMR (D₂O, ppm) : 169.0, 167.9, 53.8, 48.5, 45.3, 44.0

| | | | |
|---|---|---|---|
| Calc. for C₁₁H₁₆N₂O₁₀.2.3H₂O | 34.98%C | 5.50%H | 7.42%N |
| Found | 35.06%C | 5.76%H | 7.25%N |

### EXAMPLE 6

### Preparation of 2-Methoxypropylenediaminetetraacetic acid (MeO-PDTA)

The amino groups of 2-hydroxy-1,3-diaminopropane were protected using (BOC)₂O (quantitative yield). The product, in anhydrous tetrahydrofuran, was treated with NaH (1.1 equiv) for 1 hour, and then treated at 0°C with MeI (2 equiv), allowing the mixture to warm to ambient temperature overnight. The product was chromatographed on silica-gel, and the required product eluted with 10-20% ethyl acetate in hexane in 43% yield. It was hydrolyzed by stirring overnight at room temperature with 6N HCl, and then alkylated with bromoacetic acid at 45%, pH 9-10 overnight. The crude ligand was purified by chromatography on AG-1 X8 resin (OH- form), being eluted with 10-15N acetic acid, yield 90%.
FAB MS : MH⁺ found, 337. Calc, 337
¹H NMR (D₂O, ppm): 3.7,s, 9H : 3.3, m, 4H : 3.1 s, 3H.

### EXAMPLE 7

### Preparation of 2-Carboxymethylpropylenediaminetetraccetic acid (CMPDTA)

To malononitrile (30g, 0.45M) in anhydrous tetrahydrofuran (3000 mL) was added NaH (14.4 g, 60%. 0.36M), and the mixture stirred for 15 minutes. Benzyl bromoacetate (57 mL. 0.36M) in tetrahydrofuran (500 mL) was added slowly dropwise at 0°C, and the mixture allowed to stir overnight. The solution was filtered and the solvent evaporated to yield an orange gum. This was treated with ethanol to yield a white solid (12.5g) whose ¹H NMR spectrum indicated that it contained 93% monoalkylated malononitrile and 7% of the dialkylated product. A second treatment with ethanol improves the purity to 97%.

This product, in acetic acid solution, was reduced overnight in a Parr hydrogenator using Pearlman's catalyst, to yield 2-carboxymethyl-1,3-propylenediamine as a pale yellow gum.

FAB MS : MH⁺ found, 133, calc. 133.

This product was alkylated by the method of Example 2 (using 5 equivalents of t-butylbromoacetate since the carboxyl group is also alkylated under the reaction conditions) to yield the the ligand pentaester. This was chromatographed on silica-gel, the required product being eluted with 2-3% MeOH in CH₂Cl₂ as a colorless, viscous oil. This was hydrolysed to the ligand by stirring overnight in 6N HCl. It was purified by chromatography on AG-1 X8, the ligand being eluted with 6N HCl, and isolated as a dry, colorless foam on a rotary evaporator.

FAB MS: MH⁺ found, 365. Calc., 365.
¹H NMR (D₂O, ppm) : 3.8, 3.7, 3.0, 2.2.
¹³C NMR (D₂O, ppm) : 184, 181, 63, 61.5, 60.7, 43, 34.

### EXAMPLE 8

### Preparation of butylenediaminetetraacetic acid (BDTA)

6.0 g of 1.4-diaminobutane and 56.4g (4.25 equivalents) of t-butylbromoacetate and trisodium phosphate (77 g, 470 mmol) were combined in 200 ml acetonitrille and heated to reflux overnight with mechanical stirring. The next day, TLC of the solution showed one major spot and three very minor spots. The mixture was filtered and the salt cake was washed with further CH₃CN and discarded. The filtrate was evaporated to dryness to give a thick yellow oil which was then diluted with methanol and treated with 6N HCl (200ml). After one hour stirring, this was evaporated to dryness and treated with further 6N HCl for one an hour. The solution was evaporated to dryness, and chased once with water to give 34g solid; ¹H-NMR showed ∼98% complete deprotection. The crude t-butyl ester product was chromatographed on silica-gel, the required product being eluted with 3-4% MeOH in CH₂Cl₂.

FAB MS: MH⁺ found, 545: calculated, 545.

The ester was hydrolysed with triflic acid to yield the required ligand.

FAB MS: MH⁺ found, 321: calculated, 321.

This product was purified by dissolution in 2N NH₄OH followed by precipitation with 12N HC1.
¹H NMR (D₂O, ppm): 2.8,s, 8H; 2.2,s, 4H: 1.1,s, 4H.
¹³C NMR (D₂O, ppm): 181, 60, 56, 25.

| | | | |
|---|---|---|---|
| Calc. for C₁₂H₂₀N₂O₈,1.29HCl,1.10H₂O | 37.23%C | 6.12%H | 7.24%N |
| Found | 37.22%C | 6.06%H | 7.17%N |

### EXAMPLE 9

### Preparation of N'-methyl-DTTA (Me-DTTA)

This compound was prepared from diethylenetriamine by treatment with ethyl trifluoroacetate (2 equiv.) in CHCl₃ at 0°C, the solution then being stirred overnight. The solution was then evaporated to dryness to yield the bis (trifluoroamide) as a white solid foam. This was dissolved in dry CH₃CN, treated with MeI (1.1 equiv.) and excess solid anhydrous K₂CO₃ and stirred at room temperature for 3 hours. The mixture was filtered, the solvent evaporated, the residue dissolved in water, and the solution extracted three times with CHCl₃. The combined organic solutions were washed once with brine, dried, and evaporated to give a pale yellow oil (74% yield) which crystalized on standing. This product was deprotected by stirring overnight at room temperature in 4N NaOH, with the addition of a small amount of MeOH to clarify the solution. The solvent was evaporated, and the product amine purified by vacuum distillation, a higher boiling fraction being rejected, yield 37%. The amine was alkylated by the procedure of Example 1. The product was purified by chromatography on AG-1 X8, the desired product being eluted with 2 N acetic acid.
¹H NMR (D₂O, ppm): 3.6,s,8H; 3.3,s,8H; 2.8,s,3H.

### EXAMPLE 10

### Preparation of N-2.3-propanediol DTTA (2.3-diol-PDTA).

3-Amino-2,3-propenediol in DMF was treated with 2-bromoethyliminodiacetic acid di(t-butyl) ester (2.2 equiv.), and tetramethylguanidine (2.2 equiv.), and heated at 75°C for 24 hours. The solvent was evaporated, and the residue dissolved in CHCl₃ and extracted three times with water. The CHCl₃ was evaporated, and the residue hydrolyzed by stirring overnight in 6N HCl. The crude ligand was purified by chromatography on AG-1 X8, being eluted by 4N acetic acid.
- ¹H NMR (D₂O, ppm) :: 3.83,m,1H; 3.62,s.8H; 3.36 and 3.27,t and s 10H; 3.0,t,2H.
- ¹³C NMR (D₂O, ppm) :: : 171, 66.3, 62.8, 55.7, 55.2, 50.1, 49.7

### EXAMPLE 11

### Preparation of hydroxyethyl DTTA (HO-Et-DTTA)

Ethanolamine was stirred with 2-bromoethyliminodiacetate-t-butyl ester (2.2 equiv.), and anhydrous Na₃PO₄ (10 equiv.) in CH₃CN, and allowed to reflux overnight. The mixture was filtered and the residue chromatographed on silica-gel, the required product being eluted with 4-5% MeOH in CHCl₃. The ester was hydrolysed by stirring overnight in 6N HCl. The ligand was isolated by chromatography on AG-1 X8, the required producted being eluted with 4N acetic acid and isolated as a pale yellow gum.

FAB MS: MH⁺ found 380; calculated 380.
- ¹H NMR (D₂O, ppm) :: 3.7,t,2H; 3.6,s,8H; 3.3,t,8H; 3.1,t,2H.
- ¹³C NMR (D₂O, ppm):: 173, 56.8, 56.5, 55.6, 51.2, 50.6.

### EXAMPLE 12

### Preparation of benzyl-DTTA (Bz-DTTA)

Benzylamine was stirred with 2-bromoethyl-IDA-t-butyl ester (2.2 equiv.) and anhydrous Na₃PO₄ (10 equiv.) in CH₃CN, and allowed to reflux overnight. The mixture was filtered and the residue chromatographed on silica-gel, the required product being eluted with 3% MeOH in CHCl₃. The ester was hydrolysed by stirring overnight in 6N HCl. The ligand was isolated by chromatography on AG-1 X8, the required product being eluted with 4-5N acetic acid.
- ¹H NMR (D₂O, ppm) :: 7.1,s,5H; 3.9,s,2H; 3.3,s,8H; 3.1,s,8H
- ¹³C NMR (D₂O, ppm):: 170, 127.9, 126.9, 125.9, 59.6, 56.1, 51.7, 50.9

### EXAMPLE 13

### Preparation of N',N'-dimethyl-N,N"-diethylenetriaminetraacetic acid (DTTAO-Me).

N-methyldiethylenetriamine tetraacetic acid (prepared as in Example 9) was dissolved in MeOH, treated with 6 equiva lents of SOCl₂ dropwise, and then allowed to stir overnight. The solution was evaporated, the residue dissolved in a small amount of MeOH and a small excess of triethylamine added. The mixture was then considerably diluted with ether, and the precipitated amine hydrochloride filtered. Evaporation of the ether yielded the pure tetramethylester as a pale yellow oil in 96% yield.

The tetraester was dissolved in methanol, MeI added (1 equiv.), and the solution stirred for 1 hour at room temperature. A second addition of MeI (1 equiv.) was made, and the solution stirred for 3 days. Evaporation yielded the required quaternized product. This was hydrolyzed with 1N HC1 at 90°C for 2 hours. The crude ligand was recovered by evaporation, and purified on AG-1 X8 (acetate form). The pure ligand was eluted with 0.75N acetic acid, and evaporated several times with 1N HC1 to convert it to the hydrochloride (2HC1) salt, a white powder.
FAB MS: MH⁺ found 364. Calc. 364.
¹H NMR (D₂O, ppm) : 3.65 and 3.61,s and t, 12H; 3.3,t,4H; 3.0,s,6H.
¹³C NMR (D₂O, ppm): 171, 60.4, 57.1, 53.3, 50.2.

| | | | |
|---|---|---|---|
| Calc. for C₁₄H₂₈N₃O₈Cl₃,4.5H₂O | 30.36%C; | 6.73%H; | 7.59%N |
| Found | 30.40%C; | 6.66%H; | 7.58%N. |

### EXAMPLE 14

### Preparation of NONON-MeO

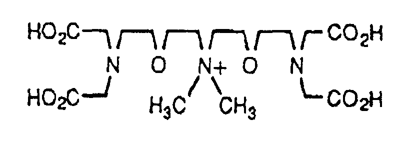

2(2-Aminoethoxy) ethanol was converted to the BOC derivative in quantitative yield with (BOC)₂O. The hydroxyl group was converted to the bromo group by treatment in CH₂Cl₂ at 0°C with triphenylphosphine (1.25 equiv.) and N-bromo-succinimide (1.25 equiv.). The bromo compound was recovered from a silica-gel column in 63% yield as a colorless oil by elution with 1:1 ether-hexane. This was used in an alkylation-quaternization reaction on dimethylamine, similar to that of Example 4 except that the mixture was refluxed for 4 days. The product was purified on silica-gel, eluting with 10% MeOH-CHCl₃, and recovered in 67% yield.

FAB MS: MH⁺ found 420. Calc. 421.

Removal of the BOC protecting groups was achieved quantitatively by treatment with 6N HCl at room temperature for 15 hours. After removal of the HCl by repeated reconcentration from water, the diamine was alkylated with bromoacetic acid under the conditions of Example 1. The product was purified by chromatography on AG-1 X8, eluted with 0.05N acetic acid as a colorless gum in 64% yield.
- ¹H NMR (D₂O, ppm) :: 3.75 and 3.69, 2s, 16H; 3.5,s,4H; 3.4,s,4H; 3.0,s,6H.
- ¹³C NMR (D₂O, ppm):: 169, 64, 56.5, 54.7, 51.8

### EXAMPLE 15

### Preparation of DTPA-cholamine:

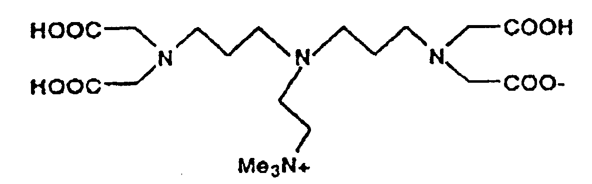

This was prepared by the method of Example 2, using 3-bromopropyliminodiacetic acid-t-butyl ester (8.05g, 22mM), cholamine hydrochloride (1.75g, 10mM), and anhydrous Na₃PO₄ (10g. 61mM), in 200mL acetonitrille, and refluxed for 4 days. The mixture was filtered and the product isolated on a rotary evaporator.

FAB MS: MH⁺ found, 674; Calc., 674.

The reaction mixture was chromatographed on silica-gel, the required product being eluted by 7.5% MeOH in CH₂Cl₂ as a yellow oil (64% yield). The ester was hydrolysed with 6N HC1 at room temperature for 1 hour. The recovered product was purified by chromatography on AG-1 X8, the product being eluted with 0.5M acetic acid (56% yield, based on cholamine)
FAB MS: MH⁺ found, 449. Calc. 449.
- ¹H NMR (D₂O, ppm) :: 3.67,s,8H; 3.55,t,2H; 3.35,t,2H; 3.15,t,4H; 3.0,s,9H; 2.95,t,4H; 1.9,t,4H
- ¹³C NMR (D₂O, ppm):: 170, 60.5, 57.8, 54.3, 54.0, 50.7, 46.5, 20.0

### EXAMPLE 18

### Preparation of the W₃(µ₃-S)₃ complex of ethylenebis-(oxyethylenenitrilo)tetraacetic acid: [Na₄((W₃(µ₃-S)(µ₂-S)₃)₂(EGTA)₃]

3.66g of [W₃(*µ*₃-S) (*µ*₂-S)₃(H₂O)₉] Cl₄ prepared as described in WO92/17215 was dissolved in 500 mL of DMF, forming a dark green solution. 2.065g of EGTA was added to the green solution. The mixture was refluxed for 4 hours until a blue suspension was obtained. After cooling the suspension, the blue solid was collected by filtration and washed with isopropanol and acetone. The yield for the crude product was 3.73g.
¹H NMR (d₆-DMSO) δ = 4.49 ppm (t, 4H), 4.19 ppm (q, 4H, J-15.81 Hz), 3.98 ppm (t, 4H), 3.60 ppm (s, 4H).

### EXAMPLE 19

### a) Preparation of [W₃SO₃(H₂O)₉] Cl₄ and [W₃S₃O(H₂O)₉]Cl₄

The title compounds were prepared by a modified procedure based on that described by Cotton, F.A., et. al. in Polyhedron 5, 907 (1986). To a three-neck flask with 24.0g of anhydrous Na₂S, 1.6mL of H₂O was added dropwise, then 40.0g of W(CO)₆, and 2.0L of acetic anhydride was added. The mixture refluxed for 6 hours under N₂. Then an additional 20g of W(CO)₆ was added and the mixture was refluxed for 16 hours. After thorough cooling of the mixture, the yellow-brown solid was dissolved in 2.0 M HCl. The red solution was filtered and the filtrate was diluted by the addition of 5 equiv. amount of water. The solution was loaded onto an AG50W-X8 cation exchange column. 0.5 M HC1 and 0.75 M HC1 was used to wash off the undesired byproducts. Elution with 4 M HCl yielded a red solution. This eluant was rotary evaporated to dryness. The resulting solid was redissolved in 2 M HC1 and loaded onto a Sephadex G-15 column. Elution with 2 M HCl yielded a red-orange band (1st band), which when evaporated to dryness at high vacuum gave a red solid [W₃SO₃(H₂O)₉]Cl₄ (15g). The second band, purple-red, was also collected and rotary evaporated to dryness under vacuum to afford a red-purple solid [W₃S₃O(H₂O)₉]Cl₄ (approx. 2g).

Elemental analysis indicated the first band was [W₃SO₃(H₂O)₉] Cl₄.

| | | |
|---|---|---|
| Calculated | W (58.95%) | S (3.43%) |
| Found | W (59.40%) | S (3.63%) |

UV-Vis: 460 nm (ε = 360 M⁻¹cm⁻¹)

The second band was identified as [W₃S₃O(H₂O)₉]Cl₄ by its UV-vis spectrum.
UV-Vis: 535 nm (ε = 410 M⁻¹cm⁻¹).

### b) Preparation of [W₃SO₃(H₂O)₉]Cl₄ and [W₃S₃O(H₂O)₉]Cl₄ with an improved ratio of W₃SO₃:W₃S₃O

To W(CO₆) (90g) and Na₂S (36g) under a blanket of N₂ was added acetic anhydride (31). The flow of N₂ was then stopped and the mixture stirred with a mechanical stirrer. The mixture was heated at reflux for 72hr. The resulting black solution was cooled to room temperature and filtered. The solid (dark brown powder and clear needles) was divided in half. Fraction A (107g, wet) was placed under vacuum and stored for future use in a freezer. The remaining Fraction B (107g, wet) was taken up in 12N HCl (1000ml) and stirred briefly. The mixture was allowed to stand for 2.5hr, then poured into H₂O (1l). The resulting solution was concentrated under vacuum at less than 50°C to 100ml. This solution was loaded onto a Sephadex (G-25, 500g) column and eluted with 2N HCl. Three distinct fractions were obtained. First was a pale orange band, second a ruby red/brown band and third a purple band. The ruby red/brown band was collected and concentrated in vacuum to dryness to yield 15.6g of the W₃SO₃ compound. In addition 3g of a 50:50 mixture of the W₃SO₃ compound and a pale orange solid and 7g of a 75:25 mixture of the W₃SO₃ compound and a purple solid were obtained.

### EXAMPLE 20

### Preparation of Na₄[(W₃SO₃)₂(EGTA)₃] and Na₄[(W₃S₃O)₂(EGTA)₃] (EGTA = Ethylene glycol bis(2-aminoethyl ether)-N.N.N'N-tetraacetic acid).

### 1. Na₄ [(W₃SO₃)₂(EGTA)₃]

5.0g of [W₃SO₃(H₂O)₉]Cl₄ was dissolved in 700 mL of DMF. Then 3.0g of EGTA was added. The mixture was refluxed for 3 hours, a red solid separated. The red solid was collected by filtration and redissolved in H₂O. It was passed through an AG50X8 column (Na⁺ form) and the resulting solution was dried by rotary evaporation. The red solid was dissolved in minimal amount of water and loaded on to a Sephadex G-25 column. Elution with water yielded three bands. The last band was collected, dried by rotary evaporation, and passed through the Sephadex G-25 column again. The third band was collected and dried to give 1.0g of red solid product. It contained 10 H₂O.
UV-Vis: 475 nm (ε = 1250 M⁻¹cm⁻¹).
- ¹H NMR (D₂O, ppm) :: 4.26 (d,4H), 4.04 (d,4H), 3.55 (t,4H) 3.44 (t, 4H), 3.35 (s, 4H).
- ¹³C NMR (D₂O) :: δ (ppm) 181.0, 70.3, 66.8, 64.1.
- ¹⁸³W NMR (D₂O, Na₂WO₄ reference) :: δ (ppm) 1206.5.
- MS-FAB⁺:: M+Na 2507.

| Elemental analysis: | | | | | |
|---|---|---|---|---|---|
| Calculated | W(41.1%) | S(2.41) | C(18.93) | H(3.03) | N(3.15) |
| | Na(3.45) | | | | |
| Found | W(39.8%) | S(2.43) | C(18.74) | H(2.88) | N(3.08) |
| | Na(3.44) | | | | |

Na₄[(W₃SO₃)₂(EGTA)₃].10H₂O was formulated as a 168 mM solution with pH 6.65; osmolality 685 mmoles/kg. The maximum tolerated dose (MTD) in mice was ∼7.75 mmoles complex/kg.

### 2. Na₄[(W₃S₃O)₂(EGTA)₃]

This compound was prepared in a similar procedure to that of Na₄[(W₃S₃O)₂(EGTA)₃] with 5.0g of [W₃S₃O(H₂O)₉]Cl₄, 800 mL of DMF, and 2.95g of EGTA. The crude product was purified by two treatments of Sephadex G-25 column. The third band was collected and rotary evaporated to dryness. 0.7g of red-purple solid was obtained. It contained 6 H₂O.
UV-Vis: 558 nm.
¹⁸³W NMR (D₂O) : δ (ppm) 2479, 1853, 1854, 1855
MS-FAB⁺: [M+Na]⁺ 2571.

| Elemental analysis: | | | | | |
|---|---|---|---|---|---|
| Calculated | W(41.53%) | S(7.20) | C(18.99) | H(2.73) | N(3.16) |
| | Na(3.46) | | | | |
| Found | W(43.12%) | S(6.60) | C(19.02) | H(2.81) | N(3.09) |
| | Na(3.84) | | | | |

Na₄[(W₃S₃O)₂(EGTA)₃] .6H₂O was formulated as a 121 mM solution with pH 6.95; osmolality 517 mmoles/kg. The maximum tolerated dose (MTD) in mice was ∼3.95 mmoles complex/kg.

### EXAMPLE 21

### Preparation of Na₄[(W₃SO₃)₂(OBETA)₃] (OBETA = Oxbis(ethylamine)-N.N.N'N'-tetraacetic acid

To a solution of 3.8g [W₃SO₃(H₂O)₉]Cl₄ in 400 mL of DMF, 1.83 g of OBETA was added. The mixture was refluxed for 3 hours, a red-orange solid separated. The solid was collected and excess amount of isopropanol was added to the filtrate, causing the separation of additonal solid. It was collected and the two batches of solids were combined. They were dissolved in H₂O and the solution was passed through a AG50X8 column (Na⁺ form). The eluant was rotary evaporated to dryness. The red solution was dissolved in minimal amount of water and loaded on to a Sephadex G-25 column. Elution with water yielded three bands. The last band was collected, rotary evaporated to dryness, and passed through the Sephadex G-25 column again. The third band was collected and rotary evaporated to dryness. 1.8g of red solid was obtained. It contained 10 H₂O.
- UV-Vis:: 475 nm
- ¹H NMR (D₂O) ;: δ (ppm) 4.28 (d, 4H), 4.17 (d, 4H), 3.55 (t, 4H), 3.39 (t, 4H)
- ¹³C NMR (D₂O) :: δ (ppm) 182.0, 67.9, 65.3
- ¹⁸³W NMR (D₂O):: δ (ppm) 1203.5
MS-FAB⁺ M+Na 2374.

| Elemental analysis: | | | | | |
|---|---|---|---|---|---|
| Calculated | W (43.56%) | S(2.53) | C(17.08) | H(2.71) | N(3.32) |
| | Na(3.60) | | | | |
| Found | W(43.77%) | S(2.70) | C(16.98) | H(2.49) | N(3.34) |
| | Na (3.18) | | | | |

### Formulation and Toxicology in Mice

Na₄[(W₃SO₃)₂(OBETA)₃].10H₂O was formulated as a 107 mM solution with pH 7.03; osmolality 498 mmoles/kg. The maximum tolerated dose (MTD) in mice was -4.35 mmoles complex/kg.

### EXAMPLE 22

### Preparation of Na₄[(W₃SO₃)₂(PDTA)₃] (PDTA = 1.3-Propylene diamine-N,N,N',N', tetraacetic acid)

To a solution of 2.0 g [W₃SO₃(H₂O)₉]Cl₄ in 160mL of DMF, 0.91 g of PDTA was added. The mixture was refluxed for 4 hr, a red solid separated. The solid was collected by filtration. It was dissolved in H₂O and the solution was passed through a AG50X8 column (Na⁺ form). The eluant was rotary evaporated to dryness. The red solid was dissolved in minimal amount of water and loaded on to a Sephadex G-25 column. Elution with water yielded three bands. The last band was collected, rotary evaporated to dryness, and passed through the Sephadex G-25 column again. The third band was collected and rotary evaporated to dryness, 1.2 g of red solid was obtained. It contained 11 H₂O.
- UV-Vis:: 475 nm.
- ¹H NMR (D₂O) :: δ (ppm) 4.20 (d, 4H), 4.09 (d, 4H), (m, 4H), 2.07 (m, 2H). 2.93
- ¹³C NMR (D₂O) :: δ (ppm) 182.6, 66.8, 63.9, 19.2.
- ¹⁸³W NMR (D₂O) :: δ (ppm) 1189
- MS-FAB⁺:: [M+H]⁺ 2262

| Elemental analysis: | | | | |
|---|---|---|---|---|
| Calculated | W (44.84%) | S (2.61) | C (16.30) | H (2.61) |
| | N (3.42) | Na (3.74) | | |
| | W (44.46%) | S (2.69) | C (16.65) | H (2.30) |
| | N (3.46) | Na (3.81) | | |

### Formulation and Toxicology in Mice.

Na₄[(W₃SO₃)₂(PDTA)₃] 11H₂O was formulated as a 79mM solution with pH 6.6; osmolality 311 mmoles/kg. The maximum tolerated dose (MTD) in mice was >6.5 mmoles complex/kg.

### EXAMPLE 23

### Preparation of Na₄[(W₃SO₃)₂(BDTA)₃] (BDTA = 1,4-Butylene diamine-N,N,N',N'-tetraacetic acid)

This compound was prepared in a similar procedure to that of Na₄[(W₃SO₃)₂(PDTA)₃], with 4.0g of [W₃SO₃ (H₂O)₉]Cl₄, 400 mL of DMF, and 2.54g of BDTA. The crude product was purified by two treatments of Sephadex G-25 column. The third band was collected and rotary evaporated to dryness. 1.1g of red solid was obtained. It contained 12 H₂O.
- UV-Vis:: 475 nm.
- ¹H NMR (D₂O) :: δ (ppm) 4.17 (d, 4H), 3.92 (d, 4H), 3.15 (b, 4H), 1.28 (b, 4H).
- ¹⁸³W NMR (D₂O) :: δ (ppm) 1209.
- MS-FAB⁺:: [M+Na]⁺ 2327.

| Elemental analysis: | | | | |
|---|---|---|---|---|
| Calculated | W (43.77%) | S (2.54%) | C(17.16) | H(2.88) |
| | N (3.33) | Na (3.65) | | |
| Found | W (44.29%) | S (2.51%) | C(16.93) | H(2.96) |
| | N (3.15) | Na (3.50) | | |

### Formulation and Toxicology in Mice

Na₄[(W₃SO₃)₂(BDTA)₃].12H₂O was formulated as a 120mM solution with pH 7.4; osmolality 459 mmoles/kg. The maximum tolerated dose (MTD) in mice was ~2.55 mmoles complex/kg.

### EXAMPLE 24

### Preparation of Na₄[(W₃SO₃)₂(HO-PDTA)₃] (HO-PDTA = 2-Hydroxy-1,3-propylene diamine-N,N,N'N'-tetraacetic acid)

To a solution of 2.0 g [W₃SO₃(H₂O)₉] Cl₄ in 100mL of DMF, 1.10 g of HO-PDTA was added. The mixture was refluxed for 3hr, a red solid separated. The DMF solvent was recovered by rotary evaporation and a red residue was obtained. It was dissolved in H₂O and NaOH was used to adjust the pH of the solution from pH 2 to pH 9. The solution was rotary evaporated to dryness. The residue was dissolved in water and filtered through a 0.22 micron filter. The pH of the solution was then adjusted back to pH 2 by the addition of HCl. Then 1.7g of [(Ph₃P)₂N]Cl (PPNCl) in warm water was added and a fine red solid separated. The solid was collected by filtration and purified by multiple recrystallization steps from MeOH/H₂O. The crystallization procedure consisted of diluting the MeOH solution of (PPN)₄[(W₃SO₃)₂(HO-PDTA)₃] with distilled water and allowing the crystals to grow slowly. The crystals were collected and washed with hot distilled water. The Na⁺ salt of the complex was obtained through cation exchange using AG50X8 resin (Na⁺ form). 0.76g of red solid was obtained. It contained 6 H₂O.
- UV-Vis:: 475 nm
- ¹H NMR (D₂O) :: δ (ppm) 4.7 (m, 1H), 4.2-4.6 (m, 8H), 3.33 (m, 2H), 2.74 (m, 2H)
- ¹⁸³W NMR (D₂O):: δ (ppm) 1207.7, 1201.3, 1196.2, 1190.7.
- MS-FAB⁺:: [M+H]⁺ 2308

| Elemental analysis: | | | | |
|---|---|---|---|---|
| Calculated | W (45.62%) | S (2.65%) | C(16.39) | H(2.25) |
| | N (3.48) | Na (3.80) | | |
| Found | W (46.05%) | S (2.68%) | C(16.02) | H(2.66) |
| | N (3.40) | Na (3.54) | | |

### Formulation and Toxicology in Mice

By neutralizing the H₄[(W₃SO₃)₂(HO-PDTA)₃] solution, which was obtained through the cation exchange using AG50X8 resin (H⁺ form), with N-methyl-d-glucamine (NMG), the (NMG)₄[(W₃SO₃)₂(HO-PDTA)₃] salt was obtained. It was formulated as a 153mM solution with pH 6.98; osmolality 499 mmoles/kg. The maximum tolerated dose (MTD) in mice was ∼2.17 mmoles complex/kg.

### EXAMPLE 25

### Preparation of Na₄[(W₃SO₃)₂(MeO-PDTA)₃] (MeO-PDTA = 2-Methoxyl-1,3-propylene diamine-N,N,N'N'-tetraacetic acid).

To a solution of 2.0 g [W₃SO₃(H₂O)₉]Cl₄ in 30mL of DMF at 150°C, a solution of 1.08g of MeO-PDTA in 30mL of DMF was added dropwise. The addition took 30 min., then the mixture was refluxed for 3 hr, a red solution separated. The solid was collected by filtration. It was dissolved in H₂O and the solution was passed through a AG50X8 column (Na⁺ form). The eluant was dried by rotary evaporation. The red solution was dissolved in minimal amount of water and loaded on to a Sephadex G-25 column. Elution with water yielded three bands. The last band was collected, rotovapped to dryness, and passed through the Sephadex G-25 column again. The third band was collected and dried by rotary evaporation. 1.4g of red solid was obtained. It contained 7 H₂O.
- UV-Vis:: 475 nm
- ¹H NMR (D₂O) :: δ (ppm) 4.2-4.6 (m, 9H), 3.71 (s, 3H) 3.33 (m, 4H), 2.8 (m, 4H)
- ¹⁸³W NMR (D₂O) :: δ (ppm) 1212.4, 1205.8, 1201.7, 1195.4,
- MS-FAB⁺:: [M+H]⁺ + 2349

| Elemental analysis: | | | | |
|---|---|---|---|---|
| Calculated | W (44.51%) | S (2.59%) | C(17.45) | H(2.52) |
| | N(3.39) | Na(3.71) | | |
| Found | W (45.13%) | S (2.35%) | C(16.77) | H(2.91) |
| | N(3.17) | Na(3.89) | | |

### Formulation and Toxicology in Mice

Na₄[(W₃SO₃)₂(MeO-PDTA)₃] . 7H₂O was formulated as a 140mM solution with pH 6.5; osmolality 563 mmoles/kg. The maximum tolerated dose (MTD) in mice was -5.25 mmoles complex/kg.

### EXAMPLE 26

### Preparation of Na₄[(W₃SO₃)₂(CM-PDTA)₃] (CM-PDTA = 2-Carboxymethyl-1.3-propylene diamine-N,N,N'N'. tetraacetic acid)

This compound was prepared in a similar procedure to that of Na₄[(W₃SO₃)₂(HO-PDTA)₃] with 2.0g of [W₃SO₃(H₂O)₉]Cl₄, 100mL of DMF and 1.21g of CM-PDTA. However, no red solid separated during the reaction, which was different to the preparation of Na₄[(W₃SO₃)₂(HO-PDTA)₃]. The Na₄[(W₃SO₃)₂(CM-PDTA)₃] was purified by multiple recrystallisation steps of the PPN⁺ salt in a similar procedure to that of Na₄[(W₃SO₃)₂(HO-PDTA)₃]. 0.50g of red solid was obtained. It contained 7 H₂O.
- UV-Vis:: 475 nm.
- ¹H NMR (D₂O):: δ (ppm) 4.1-4.6 (m, 8H), 3.7 (m, 2H), 2.5-3.0 (m, 7H),
- ¹⁸³W NMR (D₂O) :: δ (ppm) 1235.7, 1228.1, 1216.7, 1212.6.
- MS-FAB⁺:: [M+Na]⁺ 2504

| Elemental analysis: | | | | |
|---|---|---|---|---|
| Calculated | W (41.97%) | S (2.44%) | C(17.82) | H(2.26) |
| | N(3.20) | Na(6.12) | | |
| Found | W (41.70%) | S (2.62%) | C(18.22) | H(2.79) |
| | N(3.20) | Na(6.09) | | |

### EXAMPLE 27

### Preparation of Na₄[(W₃SO₃)₂(DTPA)₃] (DTPA = Diethylenetriaminepentaacetic acid)

This compound was prepared in a similar procedure to that of Na₄[(W₃SO₃)₂(HO-PDTA)₃] with 2.0g of [W₃SO₃(H₂O)₉]Cl₄, 100mL of DMF and 1.37g of DTPA. A red solid separated during the reaction, which was collected by filtration as the crude product. The Na₄[(W₃SO₃)₂(DTPA)₃] was purified by multiple recrystallisation steps of the PPN⁺ salt in a similar procedure to that of Na₄[(W₃SO₃)₂(HO-PDTA)₃]. 0.40g of red solid was obtained. It contained 5.5 H₂O.
- UV-Vis:: 475 nm (ε = 1300 M⁻¹cm⁻¹)
- ¹H NMR (D₂O):: δ (ppm) 4.24 (d, 4H), 4.02 (d, 4H), 3.37 (b, 4H), 3.01 (s, 2H), 2.93 (m, 4H)
- ¹³C NMR (D₂O) :: δ (ppm) 182.2, 180.1, 65.1, 64.4, 56.6, 51.8.
- ¹⁸³W NMR (D₂O) :: δ (ppm) 1211.6.
- MS-FAB⁺:: [M+Na]⁺ 2612

| Elemental analysis: | | | | |
|---|---|---|---|---|
| Calculated | W (41.04%) | S (2.39%) | C(18.77) | H(2.44) |
| | N(4.69) | Na(5.99) | | |
| Found | W (41.36%) | S (2.49%) | C(18.71) | H(2.97) |
| | N(4.28) | Na(6.13) | | |

### EXAMPLE 28

### Preparation of Na₄[(W₃SO₃)₂(HO-Et-DTTA)₃] (HO-Et-DTTA = N'-Hydroxyethyl-N,N,N"N"-diethylenetriaminetetraacetic acid)

This compound was prepared in a similar procedure to that of Na₄[(W₃SO₃)₂(HO-PDTA)₃] with 2.0g of [W₃SO₃(H₂O)₉]Cl₄, 100mL of DMF and 1.31g of HO-Et-DTTA. A red solid separated during the reaction, which was collected by filtration as the crude product. The Na₄ [(W₃SO₃)₂(HO-Et-DTTA)₃] was purified by multiple recrystallisation steps of the PPN⁺ salt in a similar procedure to that of Na₄[(W₃SO₃)₂(HO-PDTA)₃]. 0.10g of red solid was obtained. It contained 15 H₂O.
- UV-Vis:: 475 nm (ε = 1300 M⁻¹cm⁻¹)
- ¹H NMR (D₂O):: δ (ppm) 4.20 (d, 4H), 4.00 (d, 4H), 3.48 (b, 2H), 3.35 (m, 4H), 2.77 (m, 4H), 2.46 (b, 2H);
- ¹³C NMR (D₂O):: δ (ppm) 182.6, 65.7, 65.1, 62.0, 57.5. 53.0
- ¹⁸³W NMR (D₂O) :: δ (ppm) 1218.3.
- MS-FAB⁺:: [M+Na]⁺ 2503.

| Elemental analysis: | | | | |
|---|---|---|---|---|
| Calculated | W (40.09%) | S (2.33%) | C(18.33) | H(3.41) |
| | N(4.58) | Na(3.34) | | |
| Found | W (39.68%) | S (2.89%) | C(18.64) | H(3.01) |
| | N(4.59) | Na(3.89) | | |

### EXAMPLE 29

### Preparation of Na₄[(W₃SO₃)₂(Bz-DTTA)₃] (Bz-DTTA = N'-benzyl-N,N,N"N"-diethylenetriaminetetraacetic acid)

This compound was prepared in a similar procedure to that of Na₄[(W₃SO₃)₂(HO-PDTA)₃] with 2.0g of [W₃SO₃(H₂O)₉]Cl₄, 100mL of DMF and 1.46g of Bz-DTTA. However, only a little solid separated during the reaction, which was filtered off. The crude product was obtained by the addition of isopropanol to the filtrate. The Na₄[(W₃SO₃)₂(Bz-DTTA)₃] was purified by multiple recrystallisation steps of the PPN⁺ salt in a similar procedure to that of Na₄[(W₃SO₃)₂(HO-PDTA)₃]. 0.10g of red solid was obtained.
- UV-Vis:: 475 nm
- ¹⁸³W NMR (D₂O) :: δ (ppm) 1212.2.
- MS-FAB⁺:: [M+Na]⁺ 2643.

### EXAMPLE 30

### Preparation of Na₄[(W₃SO₃)₂(Me-DTTA)₃] (Me-DTTA = N'-methyl-N,N,N"N"-diethylenetriaminetetraacetic acid)

This compound was prepared in a similar procedure to that of Na₄[(W₃SO₃)₂(HO-PDTA)₃] with 2.0g of [W₃SO₃(H₂O)₉]Cl₄, 100mL of DMF and 1.21g of Me-DTTA. A red solid separated during the reaction, which was collected by filtration as the crude product. The Na₄[(W₃SO₃)₂(DTTA)₃] was purified by multiple recrystallisation steps of the PPN⁺ salt in a similar procedure to that of Na₄[(W₃SO₃)₂(HO-PDTA)₃]. 0.24g of red solid was obtained. It contained 10 H₂O.
- UV-Vis:: 475 nm
- ¹H NMR (D₂O) :: δ (ppm) 4.15 (d, 4H), 3.97 (d, 4H), 3.35 (b, 4H), 2.62 (b, 4H), 1.85 (s, 3H)
- ¹³C NMR (D₂O) :: δ (ppm) 182.7, 65.7, 65.3, 55.6, 43.9
- ¹⁸³W NMR (D₂O):: δ (ppm) 1212.2
- MS-FAB⁺:: [M+Na]⁺ 2413

| Elemental analysis: | | | | |
|---|---|---|---|---|
| Calculated | W (42.90%) | S (2.49%) | C(18.22) | H(3.02) |
| | N(4.90) | Na(3.58) | | |
| Found | W (42.86%) | S (2.52%) | C(18.39) | H(2.94) |
| | N(4.92) | Na(3.71) | | |

### EXAMPLE 31

### Preparation of Na₄[(W₃SO₃)₂ (Serinol-DTTA) ₃] (Serinol-DTTA = N'-Serinol-N,N,N"N"-diethylenetriaminetetraacetic acid)

This compound was prepared in a similar procedure to that of Na₄[(W₃SO₃)₂(HO-PDTA)₃] with 2.0g of [W₃SO₃(H₂O)₉]Cl₄, 100mL of DMF and 1.21g of Serinol-DTTA. A red solid separated during the reaction, which was collected by filtration as the crude product. The Na₄[(W₃SO₃)₂(Serinol-DTTA)₃] was purified by multiple recrystallisation steps of the PPN⁺ salt in a similar procedure to that of Na₄[(W₃SO₃)₂(HO-PDTA)₃]. 0.15g of red solid was obtained. It contained 14 H₂O.
- UV-Vis:: 475 nm
- ¹H NMR (D₂O) :: δ (ppm) 4.21 (d, 4H), 4.00 (d, 4H), 3.54 (d, 4H), 3.36 (b, 4H), 3.05 (b, 4H), 2.65 (m, 1H)
- ¹³C NMR (D₂O) :: δ (ppm) 182.6, 66.3, 65.8, 61.9, 48.9
- ¹⁸³W NMR (D₂O) :: δ (ppm) 1251.2
- MS-FAB⁺:: [M+Na]⁺ 2595

| Elemental analysis: | | | | |
|---|---|---|---|---|
| Calculated | W (39.07%) | S (2.27%) | C(19.14) | H(3.46) |
| | N(4.46) | Na(3.86) | | |
| Found | W (38.52%) | S (2.62%) | C(19.81) | H(3.36) |
| | N(4.51) | Na(4.35) | | |

### EXAMPLE 32

### Preparation of the cholamide derivative of the W₃SO₃-DTPA complex

The W₃SO₃DTPA complex (Example 27, 2.0g, 0.77 mM), (2-aminoethyl)trimethylammonium chloride hydrochloride (cholamine, 15.0g, 85.7 mM), 1-(3-dimethylaminopropyl)-3-ethylcarbodiimide hydrochloride (EDAC, 14.8g, 77 mM), and 1-hydroxybenzotriazole hydrate (HOBT, 11.9g, 88 mM), were stirred in water (400 mL), and the pH adjusted from 4.4 to 6.85 with 50% aqueous NaOH. The mixture was stirred for 18 hours, after which time the pH was 6.95. After concentration, this solution was passed twice through Sephadex G-10 gel filtration agent (to remove the organic reagents), the red eluate being collected in each case. This solution was next passed through ion exchange resin (Bio-Rad AG 50W-X8, Na⁺ form, to remove any traces of cholamine). This solution (pH 7.1) was evaporated to leave a dark red solid (2.03g).

### NMR Spectra

- ¹H NMR (D₂O, ppm):: 4.0, q; 3.5; 3.3; 3.2; 3.0, s[N⁺(CH₃)₃]; 2.6
- ¹³C NMR (D₂O, ppm):: 182, 176, 66.0, 65.4, 64.8, 55.8, 53.0, 35.5
- ¹⁸³W NM (D₂O, ppm) :: 1212.9

### Elemental Analysis:

The compound has the formula: C₅₇H₉₆N₁₅O₃₃W₆S₂Na. xH₂O

| Product | H% | C% | Na% | | S% | W% | HPLC purity % |
|---|---|---|---|---|---|---|---|
| Found | 4.69 | 24.34 | 8.05 | 0.34 | 2.35 | 37.35 | 95 |
| Calc., x 8.39 | 4.21 | 23.15 | 7.10 | 0.78 | 2.17 | 37.30 | |
| (x was determined thermogravimetrically) | | | | | | | |

### Osmolality:

The osmolality was determined in water and compared to the EGTA complex (Example 20).

| Complex (charge) | Concentration | Osmolality |
|---|---|---|
| EGTA (4-) | 41 mM | 199 mmole/kg |
| Cholamide | 40 mM | 79 mmole/kg |

The osmolality for the EGTA complex is as expected for a 5 particle system at this concentration. The osmolality for the cholamide complex is as expected for a 2 particle system at this concentration.

### EXAMPLE 33

### Preparation of the cholamide derivative of the W₃SO₃-CMPDTA complex

The same method of Example 32 was used for the preparation of this complex but in place of the W₃SO₃DTPA complex, the W₃SO₃-CMPDTA complex was used.

The cholamide-W₃SO₃-CMPDTA complex had the following NMR characteristics:
- ¹H NMR (D₂O, ppm):: 4.1, q; 3.6; 3.3; 3.0, d [N⁺(CH₃)₃] ; 2.6
- ¹³C NMR (D₂O, ppm) :: 183, 181, 175, 68.5, 68.0, 65.7, 64.5, 64.0, 63.5, 55.0, 34.9, 35.5
- ¹⁸³W NMR (D₂O, ppm):: 1235.7, 1228.1, 1216.7, 1212.6, in ratio 2:2:1:2.

### EXAMPLE 34

### Preparation of a hydroxyethylated analog of the W₃SO₃-DTPA complex

The method of Example 32 was used, but in place of cholamide the bis(hydroxyethylated) analog, [NH₂CH₂CH₂N⁺(CH₃)(CH₂CH₂OH)₂] [Cl⁻] was used.

This derivative had the following NMR characteristics:
- ¹H NMR (D₂O, ppm, 80°C):: 4.9, q; 4.8, 4.4; 4.3; 4.2; 3.9, S; 3.9; 3.6;
- ¹³C NMR (D₂O, ppm, 80°C) :: 181, 175, 65.7, 64.4, 63.7, 62.4, 56.5, 51.8, 51.5, 34.2.

## Claims

1. A diagnostic imaging contrast medium comprising an image contrast enhancing physiologically tolerable complex, said complex comprising a pair of interconjugated multinuclear clusters, or a physiologically tolerable salt thereof, together with at least one pharmaceutical carrier or excipient.

2. A contrast medium as claimed in claim 1 wherein the multinuclear cluster contains 3, 4, 5 or 6 metal atoms.

3. A contrast medium as claimed in claim 2 wherein the multinuclear cluster contains 3 or 4 metal atoms.

4. A contrast medium as claimed in any preceding claim wherein the multinuclear cluster contains W or Mo.

5. A contrast medium as claimed in claim 4 wherein the multinuclear cluster is a W₃ or W₄ cluster.

6. A contrast medium as claimed in any preceding claim wherein the complex is of general formula I
(M₃)₂ L₃ (I)
wherein M₃ is a multinuclear cluster containing three metal atoms and L is a ligand.

7. A contrast medium as claimed in claim 6 wherein M₃ is a cluster of formula X₃SₐO_{b} where a is 1, 2, 3 or 4, b is 0, 1, 2 or 3, a+b is 4, and X is a metal atom.

8. A contrast medium as claimed in claim 7 wherein M₃ is W₃SO₃.

9. A contrast medium as claimed in claim 6 wherein L is represented by the formula II:
(R²)₂N[(CHR⁴)ₘNR¹]ₙ(CHR⁴)ₘN(R²)₂ (II)
where each R¹ which may be the same or different represents a group R² or a C₁₋₄alkyl, phenyl-C₁₋₄alkyl, C₁₋₄hydroxyalkyl or amino-C₁₋₄ alkyl group or two R¹ groups may together represent a group CH₂CH₂NR³CH₂CH₂ where R³ is an R² group or a C₁₋₄ alkyl group optionally substituted by hydroxyl, carboxyl, aryl or amino groups;
n is 0, 1 or 2; each m is 2, 3 or 4; each R⁴ denotes a group R¹ or a carboxyl, hydroxyl or C₁₋₄alkoxy group; and each R² independently represents a hydrogen atom or an optionally amidated or esterified carboxy -(C₁₋₄ alkyl) group, wherein any amide nitrogen is substituted by group selected from hydrogen atoms and optionally hydroxylated C₁₋₄ alkyl groups.

10. A contrast medium as claimed in any preceding claim wherein the complex possesses overall zero electrical charge.

11. A contrast medium as claimed in claim 10 wherein the complex is prepared by reaction of a multinuclear cluster with a charge compensation ligand.

12. A contrast medium as claimed in claim 10 wherein the complex is prepared by post-complexation functionalization of a charged multinuclear cluster-ligand complex.

13. A contrast medium as claimed in claim 12 being the reaction product of the 2:3 W₃SO₃ complex of DTPA and/or carboxymethyl-PDTA with cholamine or N-methyl-N,N-bis(hydroxyethyl)ethylenediamine.

14. Use of a physiologically tolerable complex, said complex comprising a pair of interconjugated multinuclear clusters, or a physiologically tolerable salt thereof, for the manufacture of a contrast medium composition for use in imaging of the human or non-human animal body.

15. A method of generating an image of a human or non-human animal body which method comprises administering to said body a contrast enhancing amount of a physiologically tolerable complex, said complex comprising a pair of interconjugated multinuclear clusters, or a physiologically tolerable salt thereof, and generating an image of at least part of said body into which said agent distributes.

16. A compound of formula I or a salt thereof
(M₃)₂ L₃ (I)
wherein M₃ is a multinuclear cluster containing three metal atoms and L is a ligand.

17. A compound selected from serinol-DTTA, DTTA-Tris, N'-(polyhydroxyalkyl)-N'-methyldiethylenetriaminetetraacetic acid wherein "polyhydroxyalkyl" is CH₂CHOHCH₂OH or CH₂(CHOH)₄CH₂OH, 2-methoxypropylenediaminetetraacetic acid, 2-carboxymethylpropylenediaminetetraccetic acid, N'-methyl-DTTA, N-2,3-propanediol DTTA, hydroxyethyl DTTA, benzyl-DTTA, N',N'-dimethyl-N,N"-diethylenetriaminetraacetic acid, NONON-MeO and DTPA-cholamine

18. A process for the preparation of [W₃SO₃(H₂O)₉] Cl₄ comprising reaction of W(CO₆) and Na₂S, acidification of the reaction product with HCl and purification of [N₃SO₃(H₂O)₉] Cl₄ therefrom, **characterised in that** the reaction product is acidified with at least 6N HCl.

## Patentansprüche

1. Diagnostisches Bilderzeugungs-Kontrastmedium, umfassend einen den Bildkontrast verstärkenden, physiologisch tolerierbaren Komplex, wobei der Komplex ein Paar interkonjugierter, mehrkerniger Cluster oder ein physiologisch tolerierbares Salz hiervon, zusammen mit mindestens einem pharmazeutischen Träger oder Vehikel umfaßt.

2. Kontrastmedium nach Anspruch 1, wobei der mehrkernige Cluster 3, 4, 5 oder 6 Metallatome enthält.

3. Kontrastmedium nach Anspruch 2, wobei der mehrkernige Cluster 3 oder 4 Metallatome enthält.

4. Kontrastmedium nach irgendeinem vorangehenden Anspruch, wobei der mehrkerniger Cluster W oder Mo enthält.

5. Kontrastmedium nach Anspruch 4, wobei der mehrkernige Cluster ein W₃- oder W₄-Cluster ist.

6. Kontrastmedium nach irgendeinem vorangehenden Anspruch, wobei der Komplex die allgemeine Formel I aufweist
(M₃)₂ L₃ (I)
worin M₃ ein mehrkerniger Cluster, welcher drei Metallatome enthält, und L ein Ligand ist.

7. Kontrastmedium nach Anspruch 6, wobei M₃ ein Cluster der Formel X₃SₐO_{b} ist, worin a 1, 2, 3 oder 4 ist, b 0, 1, 2 oder 3 ist, a+b 4 ist, und X ein Metallatom ist.

8. Kontrastmedium nach Anspruch 7, wobei M₃ W₃SO₃ ist.

9. Kontrastmedium nach Anspruch 6, wobei L durch die Formel II wiedergegeben wird:
(R²)₂N[(CHR⁴)ₘNR¹]ₙ(CHR⁴)ₘN(R²)₂ (II)
worin jedes R¹ welches gleich oder verschieden sein kann, eine Gruppe R² oder eine C₁₋₄-Alkyl-, Phenyl-C₁₋₄-Alkyl-, C₁₋₄-Hydroxyalkyl- oder Amino-C₁₋₄-Alkylgruppe bedeutet, oder zwei R¹-Gruppen zusammen eine Gruppe CH₂CH₂NR³CH₂CH₂ bilden können, worin R³ eine R²-Gruppe oder eine C₁₋₄-Alkylgrupppe, wahlweise substituiert durch Hydroxyl, Carboxyl, Aryl oder Aminogruppen, ist;
n 0,1 oder 2 ist; jedes m 2, 3 oder 4 ist; jedes R⁴ eine Gruppe R¹ oder eine Carboxyl-, Hydroxyl- oder C₁₋₄-Alkoxygruppe bedeutet; und jedes R² unabhängig ein Wasserstoffatom oder eine wahlweise amidierte oder veresterte Carboxy-(C₁₋₄-Alkyl)-Gruppe bedeutet, worin ein Amidstickstoff substituiert ist durch eine Gruppe, gewählt aus Wasserstoffatomen und wahlweise hydroxylierten C₁₋₄-Alkylgruppen.

10. Kontrastmedium nach irgendeinem vorangehenden Anspruch, wobei der Komplex insgesamt elektrische Null-Ladung besitzt.

11. Kontrastmedium nach Anspruch 10, wobei der Komplex hergestellt wird durch Umsetzen eines mehrkernigen Clusters mit einem Ladungskompensierungsliganden.

12. Kontrastmedium nach Anspruch 10, wobei der Komplex hergestellt wird durch Nach-Komplexierungsfunktionalisierung eines geladenen mehrkernigen Cluster-Ligand-Komplexes.

13. Kontrastmedium nach Anspruch 12, bei dem es sich um das Reaktionsprodukt des 2:3 W₃SO₃-Komplexes von DTPA und/oder Carboxymethyl-PDTA mit Cholamin oder N-Methyl-N,N-bis-(hydroxyethyl)ethylendiamin handelt.

14. Verwendung eines physiologisch tolerierbaren Komplexes, wobei der Komplex ein Paar interkonjugierter mehrkerniger Cluster oder ein physiologisch tolerierbares Salz hiervon umfaßt, zur Herstellung einer Kontrastmediumzusammensetzung zur Verwendung bei der Bilderzeugung des menschlichen oder nicht menschlichen tierischen Körpers.

15. Verfahren zur Erzeugung eines Bildes eines menschlichen oder nicht menschlichen tierischen Körpers, welches Verfahren das Verabreichen an den Körper einer kontraststeigernden Menge eines physiologisch tolerierbaren Komplexes, wobei der Komplex ein Paar interkonjungierter mehrkerniger Cluster oder ein physiologisch tolerierbares Salz hiervon umfaßt, und das Erzeugen eines Bildes mindestens eines Teils des Körpers, in welchem sich das Mittel verteilt, umfaßt.

16. Verbindung der Formel I oder ein Salz hiervon
(M₃)₂ L₃ (I)
worin M₃ ein mehrkerniger Cluster, welcher drei Metallatome enthält, und L ein Ligand bedeutet.

17. Verbindung, gewählt aus Serinol-DTTA, DTTA-Tris, N'-(Polyhydroxalkly)-N'-methyl-diethylentriamintetraessigsäure, worin "Polyhydroxyalkyl" CH₂CHOHCH₂OH oder CH₂(CHOH)₄CH₂OH bedeuted, 2-Methoxypropylendiamintetraessigsäure, 2-Carboxymethylpropylendiamintetraessigsäure, N'-Methyl-DTTA, N-2,3-Propandiol-DTTA, Hydroxyethyl-DTTA, Benzyl-DTTA, N',N'-Dimethyl-N,N"-diethlyentriamintetraessigsäure, NONON-MeO und DTPA-Cholamin.

18. Verfahren zur Herstellung von [W₃SO₃(H₂O)₉]Cl₄, umfassend die Umsetzung von W(CO₆) und Na₂S, Acidifizieren des Reaktionsprodukts mit HCl und Reinigen von [W₃SO₃(H₂O)₉]Cl₄ daraus, **dadurch gekennzeichnet, daß** das Reaktionsprodukt mit mindestens 6N HCl acidifiziert wird.

## Revendications

1. Agent de contraste pour imagerie de diagnostic comprenant un complexe, physiologiquement tolérable, activant le contraste de l'image, ledit complexe comprenant une paire d'agglomérats polynucléaires interconjugués, ou sel physiologiquement tolérable de celui-ci, avec au moins un support ou excipient pharmaceutique.

2. Agent de contraste selon la revendication 1, dans lequel l'agglomérat polynucléaire contient 3, 4, 5 ou 6 atomes de métal.

3. Agent de contraste selon la revendication 2, dans lequel l'agglomérat polynucléaire contient 3 ou 4 atomes de métal.

4. Agent de contraste selon l'une quelconque des revendications précédentes, dans lequel l'agglomérat polynucléaire contient W ou Mo.

5. Agent de contraste selon la revendication 4, dans lequel l'agglomérat polynucléaire est un agglomérat de W₃ ou W₄.

6. Agent de contraste selon l'une quelconque des revendications précédentes, dans lequel le complexe répond à la formule générale I
(M₃)₂L₃ (I)
dans laquelle M représente un agglomérat polynucléaire contenant 3 atomes de métal et L représente un ligand.

7. Agent de contraste selon la revendication 6, dans lequel M₃ représente un agglomérat de formule X₃SₐO_{b}, dans laquelle a est égal à 1, 2, 3 ou 4, b est égal à 1, 2 ou 3, a + b est égale à 4, et X représente un atome de métal.

8. Agent de contraste selon la revendication 7, dans lequel M₃ représente W₃SO₃.

9. Agent de contraste selon la revendication 6, dans lequel L est représenté par la formule II :
(R²)₂N[(CHR⁴)ₘNR¹]ₙ(CHR⁴)ₘN(R²)₂ (II)
dans laquelle chacun des R¹ qui peuvent être identiques ou différents, représente un groupe R² ou un groupe alkyle en C₁₋₄, phényl-alkyle en C₁₋₄, hydroxyalkyle en C₁₋₄, ou aminoalkyle en C₁₋₄, ou deux groupes R¹ peuvent représenter ensemble un groupe -CH₂CH₂NR³CH₂CH₂- dans lequel R³ représente un groupe R² ou un groupe alkyle en C₁₋₄ éventuellement substitué par des groupes hydroxyle, carboxyle, aryle ou amino ; n est égal à 0, 1 ou 2 ; chaque m est égal à 2, 3 ou 4; chaque R⁴ représente un groupe R¹ ou un groupe carboxyle, hydroxyle, ou alcoxy en C₁₋₄ ; et chaque R² représente indépendamment un atome d'hydrogène ou un groupe carboxy(alkyle en C₁₋₄) éventuellement amidifié ou estérifié, dans lequel tout atome d'azote d'un groupe amide est substitué par un groupe choisi parmi des atomes d'hydrogène et des groupes alkyles en C₁₋₄ éventuellement hydroxylés.

10. Agent de contraste selon l'une quelconque des revendications précédentes, dans lequel le complexe possède une charge électrique globale nulle.

11. Agent de contraste selon la revendication 10, dans lequel le complexe est préparé par réaction d'un agglomérat polynucléaire avec un ligand de compensation de charge.

12. Agent de contraste selon la revendication 10, dans lequel le complexe est préparé par introduction de groupes fonctionnels, postérieurement à la formation du complexe, dans un complexe ligand-agglomérat polynucléaire chargé.

13. Agent de contraste selon la revendication 12, qui est le produit de réaction du complexe 2:3 W₃SO₃ du DTPA et/ou du carboxyméthyl-PDTA avec la cholamine ou la N-méthyl-N,N-bis(hydroxyéthyl)éthylènediamine.

14. Utilisation d'un complexe physiologiquement tolérable, ledit complexe comprenant une paire d'agglomérats polynucléaires interconjugués, ou un sel physiologiquement tolérable de celui-ci, pour la fabrication d'une composition d'un agent de contraste destinée à être utilisée pour la formation d'une image du corps humain ou animal.

15. Procédé de formation d'une image d'un corps humain ou animal, ce procédé comprenant l'administration audit corps d'une quantité activant le contraste d'un complexe physiologiquement tolérable, ledit complexe comprenant une paire d'agglomérats polynucléaires interconjugués, ou d'un sel physiologiquement tolérable de celui-ci, et la formation d'une image d'au moins une partie dudit corps dans lequel ledit agent se distribue.

16. Composé de formule I ou un sel de ce dernier
(M₃)₂L₃ (I)
dans laquelle M représente un agglomérat polynucléaire contenant 3 atomes de métal et L représente un ligand.

17. Composé choisi parmi le sérinol-DTTA, le DTTA-Tris, un acide N'-(polyhydroxyalkyl)-N'-méthyldiéthylènetriaminetétraacétique dans lequel le groupe «polyhydroxyalkyle» est un groupe -CH₂CHOHCH₂OH ou -CH₂(CHOH)₄CH₂OH, l'acide 2-méthoxypropylènediaminetétraacétique, l'acide 2-carboxyméthylpropylènediaminetétraacétique, le N'-méthyl-DTTA, le N-2,3-propanediol-DTTA, l'hydroxyéthyl-DTTA, le benzyl-DTTA, l'acide N',N'-diméthyl-N-N"-diéthylènetriaminetétraacétique, le NONON-MeO, et la DTPA-cholamine.

18. Procédé de préparation du [W₂SO₃(H₂O)₉]Cl₄ comprenant les étapes consistant à faire réagir W(CO₆) et Na₂S, à acidifier le produit de réaction à l'aide de HCl et à purifier le [W₂SO₃(H₂O)₉]Cl₄ ainsi obtenu, **caractérisé en ce que** l'on acidifie le produit de réaction avec de l'HCl d'au moins 6N.
